# EUROPEAN PATENT SPECIFICATION

(11) **EP 2 007 329 B1**
(45) Date of publication and mention of the grant of the patent: **17.07.2013**
(21) Application number: 07732342.6
(22) Date of filing: 03.04.2007
(51) Int. Cl.: A61F 5/08

(54) **NASAL DILATOR**
NASENDILATATOR
DILATATEUR NASAL

(30) Priority: 03.04.2006 GB 0606669
(43) Date of publication of application: 31.12.2008
(73) Proprietor: Stewart, Keith, Port Seton East Lothian EH32 0EZ (GB)
(72) Inventor: Stewart, Keith, Port Seton East Lothian EH32 0EZ (GB)
(74) Representative: Williams, Bryn David
(86) International application number: PCT/GB2007/001298
(87) International publication number: WO 2007/113586

(56) References cited:
- EP-A- 0 958 798
- WO-A-99/55404
- FR-A1- 2 419 711
- US-A- 5 931 852

## Description

### Field of the Invention

The present invention relates to a nasal dilator, in particular but not exclusively, to nasal dilators that can improve inhalation and exhalation through the nose and thereby alleviate problems associated with chronic or temporary nasal congestion. Additionally, the invention relates to methods of inserting the nasal dilator into the nose and the treatment of chronic or temporary nasal congestion.

### Background to the Invention

Mucus is a viscous colloid made by goblet cells in the mucous membranes of the nose. Mucus contains antiseptic enzymes (such as lysozyme), immunoglobulins, mucins and inorganic salts suspended in water. In the respiratory system, it catches foreign matter and tries to prevent it from entering the body. Mucus is normally secreted in only one nostril at a time, and the nostril secreting the mucus changes about every 4 to 6 hours. Although the secretion of mucus has important physiological functions, abnormally increased mucus production in the respiratory tract is problematic and is a symptom of many common diseases, such as the common cold. The presence of mucus in the nose and throat is normal, but increased quantities can impede comfortable breathing.

Nasal congestion is the blockage of the nasal passages and is usually due to excessive secretion of mucus. As mucus is usually secreted in one nostril at a time, it is normal to have only a single nostril blocked at any one time. Nasal congestion has many causes and can range from a mild annoyance to a life-threatening condition. Nasal congestion can interfere with the ears, hearing, and speech development. Significant congestion may interfere with sleep, cause snoring, can be associated with sleep apnea, can result in insufficient oxygen levels and hypoxia, as well as right-sided heart failure. A blocked nose can also causes mild facial and head pain.

The anatomical structure of the human nose contributes to the difficulties experienced when breathing. Specifically, the lower internal portion of each nostril, known as the vestibule, typically tapers inward to a narrowed area, known as the ostium intemum, which defines the opening of the nasal air passage defined by the isthmus nasi (running from 1.65 to 2.65 cm from the nostril, and is typically about 0.3 cm in diameter). It is this narrowed portion of the nose that can become blocked with mucus. The ostium internum (located about 1 to 1,5cm from the nostril) is an orifice whose pear-shaped format, visualized under anterior rhinoscopy, is laterally limited by the inferior border of lateral superior cartilage, medially to the nasal septum and inferiorly to the floor of the nasal cavity. Chemical decongestants, such as pseudoephidrine and oxymetazoline, are commonly used to clear nasal decongestion. These chemicals work by reducing inflammation of the nasal cavity and acting as vasoconstrictors. However, where possible, it is not desirable to use such chemical interventions as they can be associated with harmful side affects.

Cage or stent like devices have also been used to combat nasal decongestion, see for example US Patent No. 4,414,977. Such devices work by acting as fixed volume dilators and are placed inside the nostril. However, these dilators are not capable of expanding the breadth of the nasal cavity passed the fixed volume, are uncomfortable to wear, and difficult to retrieve after use.

Nasal dilators are known that include a pair of outwardly biased opposing limbs that are designed for inserting one limb into each nostril (see, for example, British Patent Publication No. 2 330 079). Such devices expand the outermost fleshy portion of the vestibule of the nose which can be uncomfortable and may result in a transient or even permanent stretching of that tissue, which can be undesirable for cosmetic reasons. Additionally, as both nostrils are being dilated at the same time, each nostril cannot be optimally dilated. There is a finite amount of tissue forming the nostril, which is capable of a finite amount of stretch, and so if both nostrils are being stretched in two opposing directions optimum dilation of each nostril is not possible.

WO 99/55404 describes a device for inserting into the human nose. The device is characterised by at least one elastically deformable, plate-like base element with an inside and an outside and an essentially rectangular shape. In the device's unstressed state, said base element is essentially flat or slightly bent.

FR2419711 describes a nasal dilator for insertion in a nostril. The nasal dilator comprises two opposing limbs joined at a bridge. The nasal dilators are used in pairs, each nasal dilator being inserted, bridge first, into a nostril such that the ends of the limbs dilate the vestibule area of the nostril.

### Summary of the Invention

It is therefore an object of the invention to provide a nasal dilator that is capable of alleviating breathing problems associated with nasal congestion, but that does not possess the problems associated with the prior art, as discussed above.

According to a first aspect of the present invention there is provided a nasal dilator comprising at least two opposing limbs that are biased apart and that are configured so as to be capable of being inserted simultaneously into a single nostril.

It has surprisingly been found that inserting such a dilator into the nostril of the nasal passage that is unblocked can provide the optimum dilation of the nasal passage that cannot be achieved by a dilator that, for example, dilates both nasal passages simultaneously. Dilation of the soft tissue of the vestibule does not provide any benefit so the nasal dilator is preferably configured such that once both limbs are inserted they may perform an outward dilation of the nostril at the soft tissue surrounding the ostium internum.

The applicant has found that one of the simplest ways in which to construct such a dilator is by connecting the opposing limbs by a resiliently deformable bridge. Such a construction may be created by moulding (e.g. injection moulding) the device so as to leave the mould with the at least two opposing limbs connected by a resiliently deformable bridge configuration.

Preferably, the dilator is arranged such that the deformable bridge is adjacent an end of each limb.

Preferably, the limbs extend from the bridge in substantially the same direction.

Most preferably, the limbs diverge as they extend away from the bridge. Divergence of the limbs ensures the optimum dilation of the nasal passage without extending the nostril entrance.

Alternatively, the dilator may be formed from a length of material comprising a notch, around which the length of material may be bent so as to form the resiliently deformable bridge. In one embodiment, the length of material extending from the notch defining the limbs. Such simple constructions have the advantage of being cheap and easy to manufacture.

In order to configure the dilator so as to be inserted easily into the nose, the thickness of the limbs taper from the thickest point at the bridge. Preferably the diameter of each limb at its thickest point is less than 0.3, 0.25, 0.2 or 0.1 cm. All limbs are preferably of equal length, and so maintain equal pressure on the nasal cavity from each limb and so prevent any torsional forces. The applicant has found that in order for the limbs to extend a sufficient distance into the nasal cavity so as to dilate the tissue around the ostium internum each limb is preferably more than 1, 1.5, 2 or 2.5 cm in length.

For comfort of the user it is also preferred that the nasal dilator further comprises a cushioned portion provided on each limb. The cushioned portions may be provided, at or towards the end of each limb that is furthest removed from the bridge. It has been found that cushioned portions that mould or are shaped to the contours of the nasal cavity are most comfortable, for example concaved pads. Concaved pads are preferred as they match the contours of the nasal cavity and provide only limited obstruction to the airway. The cushioned portions may be provided directly onto the limbs or may provided on a stalk that laterally extends from the end of the limb that is furthest removed from the bridge. The stalk may be off reduced thickness to the rest of the limb so that the outer surface of the limb and the cushioned portion can be continuous. The cushioned portion maybe sleeves. The cushioned portions may be bonded to the stalks or limbs or maybe moulded onto the stalks or limbs. The stalks or limbs may include a projecting portion to retain the cushioned pads thereon.

The applicant has found that it may be desirable to include a feature of the invention that prevents the device from being inhaled or pushed too far into the nasal cavity. Thus, the dilator may include at least one stop means associated with at least one limb and configured such that the dilator cannot be inserted into the nasal cavity passed the stop means.

The stop means preferably extends laterally from at least one limb. The stop means may alternatively be formed by at least one laterally extending kink in the at least one limb. In such a confirmation the bend or kink along the otherwise substantially straight limb can block the insertion of the dilator passed the kink in the limb. Two kinks may be provided on one limb with a finger grip portion defined by the portion of the limb provided between the kinks. Alternatively, the stop means may also act as a finger grip portion. In the interests of cheap and efficient manufacture the stop means may be formed integrally with at least one limb. In order to provide the required blocking, the stop means preferably extends more than 1 cm from the limb, or the kink extends more than 1 cm from the line of the limb. It is preferred that each limb extends more than 1.5, 2 or 2.5 cm from the stop means to the end of each limb that is furthest removed from the bridge. Preferably the same stop means is provided in all limbs. An alternative or additional safety feature that could be included in the present invention is an elbow bend in at least one limb. Such a bend in the limb prevents the insertion of the dilator passed the point in the limb of the bend. Preferably the elbow defines an angle in the limb of substantially 90° and/or the same elbow bend is provided in all limbs.

The applicant has found that in order to spread the outward pressure from the dilator and optimise the dilation of the nasal cavity the dilator may be provided with a plurality of limbs, for example, 2, 3, 4, 5 or more limbs. The limbs are preferably equally biased away from each other.

Embodiments of the present invention may be configured for use during the day, when a device extending from the nose is not desired, for cosmetic reasons. Thus, the limbs and resiliently deformable bridge may be configured, when in use, so as to be entirely housed within the nasal cavity. Such configurations may be achieved in a number of ways, however, the inventor has found the following two embodiments to be particularly effective. The resiliently deformable bridge may take the form of a horse shoe with each limb extending in a perpendicular manner therefrom. The horse shoe shaped bridge may be shaped so as to mirror the shape of the internal nasal cavity. Alternatively, the resiliently deformable bridge may be formed from a first and second resiliently deformable u-bend that are connected by a third resiliently deformable u-bend, wherein the third resiliently deformable u-bend is inverted in relation to the first and second deformable u-bends, i.e. the resiliently deformable bridge takes a "M" shape.

In a further preferred embodiment of the present invention, the nasal dilator may comprise a passageway through which air can flow. In this way, even if the dilator is inserted into a blocked nasal cavity the passageway may pass through the blockage and the limbs help to expand the area for the passageway to pass through. The passageway may be associated with the nasal dilator in a number of ways, for example, the passageway can be associated with a limb from the bridge or attached directly to the bridge. The passageway may be a tubular member. Such a tubular member can be moveably retained on a limb. In order to improve the ease in which such a dilator may be inserted the passageway can be rotatably mounted and/or made of a flexible material and/or a mesh. As the passageway may need to pass further into the nasal cavity than the opposed limbs, possibly even into the isthmus nasi, the limb associated with the passageway may extend further from the bridge than the other limbs. In order to prevent the passageway from becoming blocked, it preferably includes a mesh provided at its end furthest removed from the bridge.

The dilator should be made of a light material so that it may be easily worn in the nose without causing any discomfort. The nasal dilator is preferably less than 1, 0.5 or 0.25 g in weight. The nasal dilator may be made from synthetic plastics, such as nylon, polyethylene, polypropylene, or any combination thereof. Such a dilator can be cheaply made by integral moulding.

In a preferred embodiment, the nasal dilator comprises acetal, most preferably medical grade acetal. Acetal is advantageous because it has good shape memory and nasal dilators of specific tensions can be manufactured. Acetal is also very resistant to bending fatigue.

In an alternative embodiment, the nasal dilator comprises polycarbonate. Polycarbonate lends itself to being adjusted by the user to a preferred tension.

In order to prevent the need to wash the dilator or risk the transfer of infection the dilator is preferably disposable.

In a second aspect of the present invention, there is provided a method of inserting a nasal dilator comprising the steps of urging together opposing limbs of a dilator that comprises at least two biased apart limbs so as to reduce the profile of the dilator, passing all limbs of the reduced profile dilator in through a single nostril, and releasing the limbs such that they spring apart providing an outward dilation of the single nostril.

The dilator is preferably passed through the nostril until they may perform an outward dilation of the nostril at the soft tissue surrounding the ostium internum.

Preferably, there are two limbs.

Preferably, the step of passing the limbs in through a single nostril comprises inserting the limbs such that a plane through the limbs is substantially parallel to the nasal septum.

Preferably, the step of passing the limbs in through a single nostril comprises rotating the nasal dilator such that the plane through the limbs is substantially perpendicular to the septum.

Preferably, once the limbs are released the limbs outwardly dilate a side of the nostril away from the septum.

The method may include a first step of locating the unblocked nostril for inserting the dilator into. This can simply be achieved by the user closing one nostril and exhaling through the open nostril, if exhalation is difficult then the open nostril is congested. Alternatively, the method may involve inserting one dilator into each nostril.

The method may be used to maintain an open airway and may therefore make it easier for the user to achieve the state of sleep. The method may be used to treat nasal congestion.

Preferably the dilator used in the second aspect of the present invention is any one of the dilators described in the first aspect of the present invention.

It will be understood that any of the features of the first aspect may be applicable to the second aspect and are not repeated for brevity.

### Brief Description of the Drawings

The present invention will now be described, by way of example, with reference to the accompanying drawings, in which:-
Figure 1 shows an embodiment of the present invention with two limbs.
Figure 2 shows an embodiment of the present invention with three limbs.
Figure 3 shows an embodiment of the present invention with two non-linear limbs.
Figure 4 shows an embodiment of the present invention with three non-linear limbs.
Figure 5 shows an embodiment of the present invention with an elbow bend.
Figure 6 shows an embodiment of the present invention with a horse shoe shaped resiliently deformable bridge.
Figure 7 shows an embodiment of the present invention with an "M" shaped resiliently deformable bridge.
Figure 8 shows an embodiment of the present invention with a passageway through which air may flow.
Figure 9 shows an embodiment of the present invention with combined spars and grip portions.
Figure 10 shows an embodiment of the present invention with a notched bridge.
Figure 11 shows an embodiment of the present invention with a reduced profile.

### Detailed Description of the Drawings

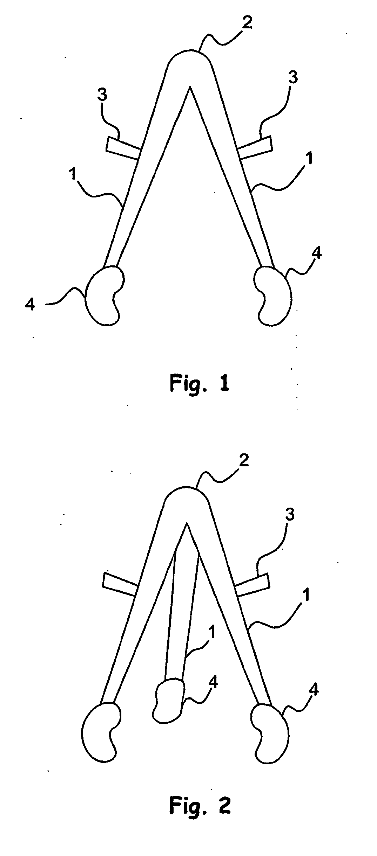

The nasal dilator of the present invention, as illustrated in Figure 1, comprises a pair of opposing limbs (1) that are connected by a resiliently deformable bridge (2). A spar (3) extends laterally from each limb (1) and concave pads (4) are provided at the end of each limb (1) that is furthest removed from the bridge (2). In use, the spars (3) engage with the outer portion of the nostril during insertion of the dilator into the nostril, thereby preventing insertion passed the point on the limb (1) from where the spars (3) extend.

A further embodiment of the nasal dilator of the present invention can be seen in Figure 2. This embodiment is similar to the embodiment shown in figure 1, but has three limbs (1). Each limb (1) is equally biased, and so spaced, apart.

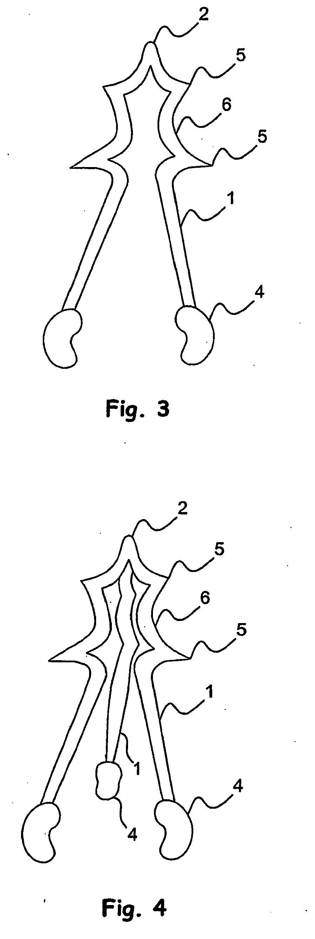

A further embodiment of the nasal dilator of the present invention can be seen in Figure 3. This embodiment is similar to the embodiment shown in Figure 1, but instead of including a spar (3), each limb includes two kinks (5) at the portion of each limb that is closest to the bridge (2). Between these kinks (5) is defined a grip portion (6) that is sized so as to be suitable to accommodate a finger tip. In use, the user may grip the dilator between two fingers, each finger resting in the grip portion (6). The kinks (5) engage with the outer portion of the nostril during insertion of the dilator into the nostril, thereby preventing insertion passed the point on the limb (1) from where the kink (5) that is furthest removed from the bridge (2) is formed.

A further embodiment of the nasal dilator of the present invention can be seen in Figure 4. This embodiment is similar to the embodiment shown in figure 3, but has three limbs (1). Each limb (1) is equally biased, and so spaced, apart.

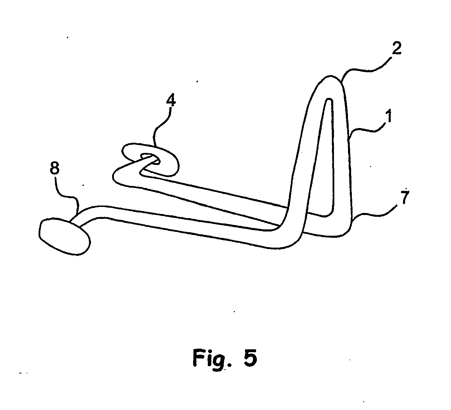

A further embodiment of the nasal dilator of the present invention can be seen in Figure 5. In this embodiment the limbs (1) include identical 90 degree bends (7) in the portion of each limb (1) towards the bridge (2). The limbs (1) include protrusions (8) that laterally extend from the end of each limb (1) that is furthest removed from the bridge (2). The protrusions (8) laterally extend away from the protrusion (8) on the opposing limb (1). Concave pads are provided at the end of each lateral protrusion. In use, the portion of the limb (1) that extends from the 90 degree bend (7) to the bridge (2) engages with the outer portion of the nostril during insertion of the dilator into the nostril, thereby preventing insertion passed the 90 degree bend (7). In use the outward bias of the limbs (1) urges the concave pads (4) towards the soft tissue within the nasal cavity that surrounds the ostium internum.

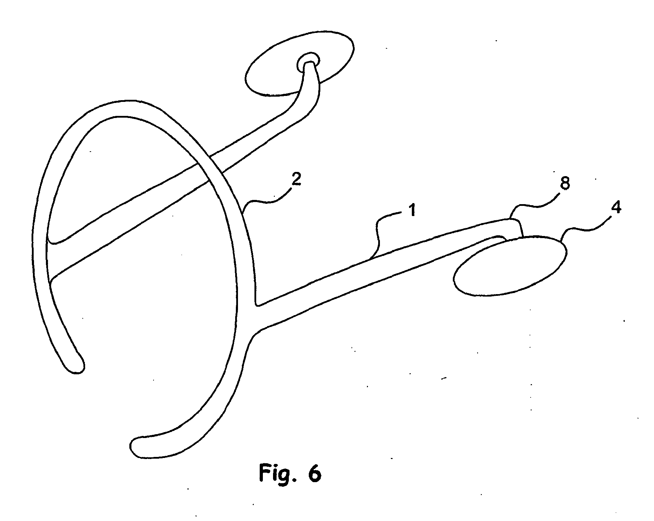

A further embodiment of the nasal dilator of the present invention can be seen in Figure 6. In this embodiment the deformable bridge (2) is not formed from a u-bend, like earlier embodiments, but is instead provided in the shape of a horse shoe. A limb (1) extends in a perpendicular manner from the horse shoe shaped bridge (2) at each of the portions provided towards the free ends of the horse shoe (2). As the horse shoe bridge (2) is resiliently deformable, in use, it may be squeezed between two fingers, thereby narrowing the profile of the horse shoe (2) and bringing the limbs (1) closer together. In such a squeezed configuration, the dilator may easily pass entirely into the nasal cavity. When the pressure applied to the bridge (2) is removed, once the dilator has been inserted into the nasal cavity, the bridge (2) is able to return to its original shape which conforms to the shape of the interior of the nasal cavity and the outward bias of the limbs (1) urges the concave pads (4) towards the soft tissue within the nasal cavity that surrounds the ostium internum.

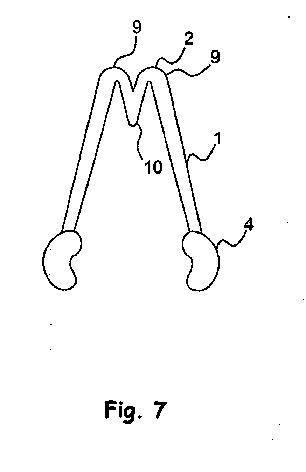

A further embodiment of the nasal dilator of the present invention can be seen in Figure 7. In this embodiment the deformable bridge (2) is formed from a first and second resiliently deformable u-bend (9)that are connected by a third resiliently deformable u-bend (10), which is inverted in relation to the first and second deformable bridge (10). Thus, the bridge (2) takes the form of an "M".

In the previous two embodiments of the present invention, the configurations permit the dilator to be entirely housed within the nasal cavity.

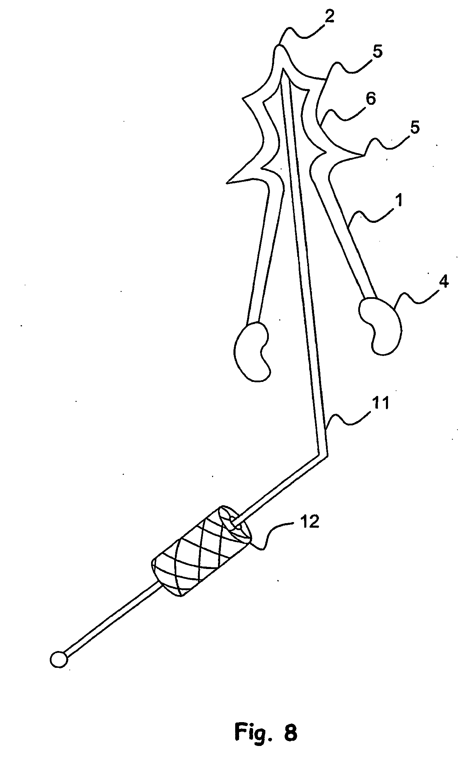

A further embodiment of the nasal dilator of the present invention can be seen in Figure 8. This embodiment is similar to the embodiment illustrated in Figure 3, but includes a longer limb (11) that extends further than the other two limbs (1). This longer limb (11) includes a tube (12) rotatably mounted around a portion of the longer limb (11). The tube (12) is made from a mesh. In use, the dilator can be inserted into the nostril in much the same manner as described for Figure 3 above, however, the third limb (11) is inserted passed the ostium internum and into the isthmus nasi. When inserted, the tube is positioned in the isthmus nasi and forces the walls of this cavity apart thereby defining a passageway through which air can travel. This is assisted by the outwardly biased limbs (1). The longer limb (11) and the tube (12) are flexible, enabling them to be configured to the shape of the nasal passageway. The tube (12) can move longitudinally and rotate on the third limb (11) so as to be positioned in at the appropriate position along the longer limb (11).

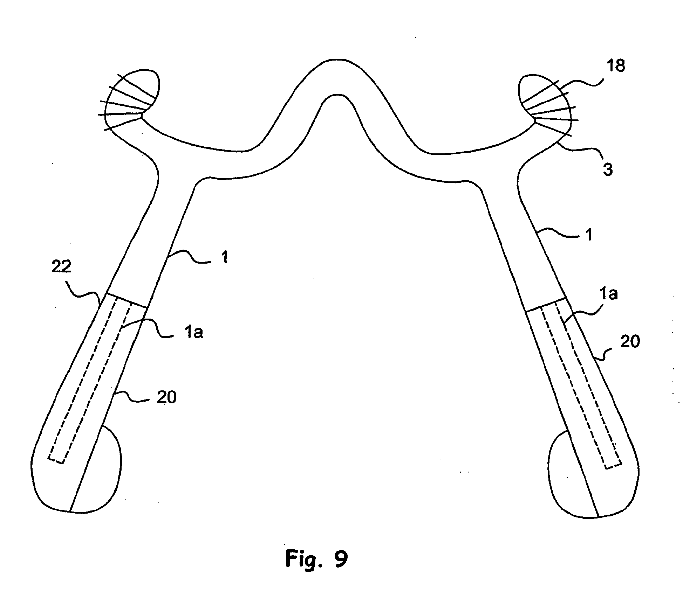

Figure 9 shows a still further embodiment of the present invention with combined spars (3) and grip portions (18). In this embodiment each limb (1) defines a reduced lower limb section (1a, shown in broken outline) over which cushioned sleeves (20) can be positioned. The cushioned sleeves (20) are moulded out of a soft elastomer and are bonded to the reduced limb sections (1a) to provide a continuous outer limb surface (22). The cushioned sleeves (20) define a rounded end (24) to assist in easing the dilator into the nasal cavity with minimum discomfort to the wearer.

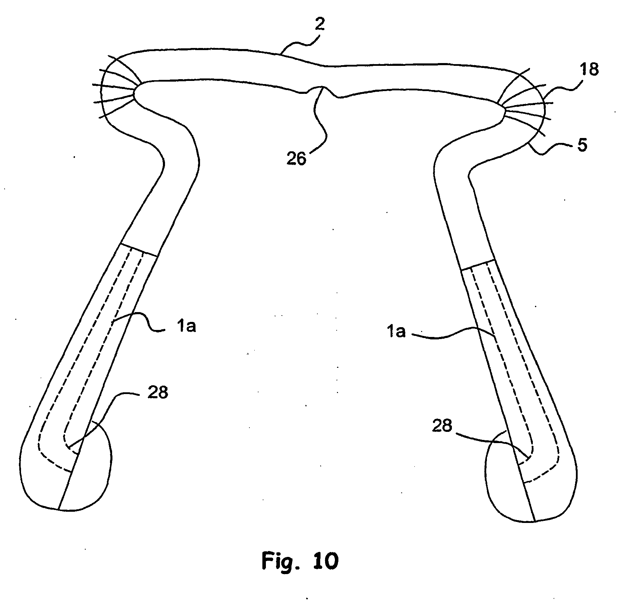

Figure 10 shows an embodiment of the present invention with a notched bridge (2). The bridge (2) of the dilator bends around a notch (26) located at the midpoint of the bridge (2). The bridge (2) also includes opposing kinks (5) which prevent over insertion over the dilator into the nasal cavity. As can be seen from Figure 10, the kinks (5) also define grip portions (18). In this embodiment, the lower ends of the reduced limb portions (1a) define projections (28). Rather than being moulded separately, cushioned sleeves (20) are moulded over the lower limb portions (1a), the projections (28) being provided to prevent the cushioned sleeves (20) from slipping off.

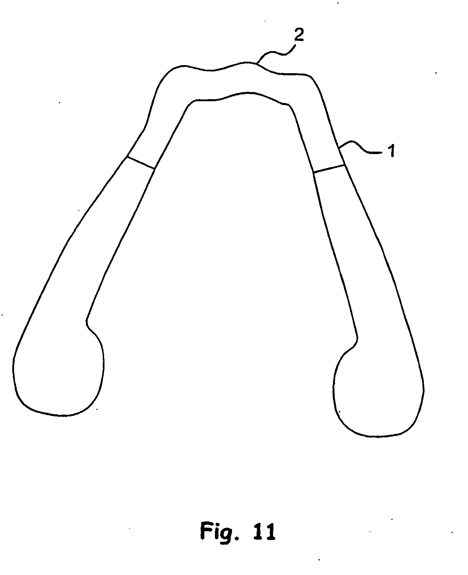

Figure 11 shows an embodiment of the present invention with a reduced profile. In this embodiment, the bridge (2) is fairly flat so that the dilator can be inserted fully into the nose and worn more discreetly.

When using the above devices, the user will first determine which one of the nasal passageways that is blocked by physically closing one nostril by pinching it with ones fingers and exhaling through the remaining open nasal passageway. If it is difficult to exhale through the open passageway then this passageway is congested. If exhalation is easy then this is the nostril in which the dilator is to be inserted in the above mentioned manner.

The present invention has been described above purely by way of example. It should be understood that modifications in detail may be made within the scope of the invention as defined in the appended claims.

## Claims

1. A nasal dilator configured to be inserted into a single nostril, the nasal dilator comprising at least two opposing limbs (1) that are biased apart and that are configured so as to be capable of being inserted simultaneously into a single nostril, wherein once said limbs (1) are inserted they perform an outward dilation of the nostril at the soft tissue surrounding the ostium internum, **characterized in that** the opposing limbs (1) are connected by a resiliently deformable bridge (2), the thickness of the limbs (1) taper from the thickest point at the bridge (2).

2. A nasal dilator as claimed in claim 1, wherein the dilator is moulded so as to form at least two opposing limbs (1) connected by the deformable bridge (2).

3. A nasal dilator as claimed in claim 1, formed from a length of material comprising a notch, around which the length of material may be bent so as to form the resiliently deformable bridge (2) with the lengths of material extending from the notch defining the limbs (1).

4. A nasal dilator as claimed in any of the preceding claims, further comprising a cushioned portion provided on each limb (1), wherein the cushioned portion is provided at or towards the end of each limb (1) that are furthermost removed from the bridge (2).

5. A nasal dilator as claimed in any of the preceding claims, wherein the limbs (1) and resiliently deformable bridge (2) are configured, when in use, so as to be entirely housed within the nasal cavity.

6. A nasal dilator as claimed in claim 5, wherein the resiliently deformable bridge (2) is formed from a first and second resiliently deformable u-bend (9) that are connected by a third resiliently deformable u-bend (10), wherein the third resiliently deformable bridge (2) is inverted in relation to the first and second deformable bridge (2).

7. A nasal dilator as claimed in any of claims 1 to 4, further comprising at least one stop means associated with at least one limb (1) and configured such that the dilator cannot be inserted into the nasal cavity past the stop means.

8. A nasal dilator as claimed in claim 7, wherein at least one stop means is formed integrally with the at least one limb (1) and wherein the stop means is formed from-a laterally extending kink (5) in at least one limb (1).

9. A nasal dilator as claimed in any of the preceding claims, wherein each limb (1) is more than 1, 1.5, 2 or 2.5 cm in length and when dependent upon claims 7 to 8 each limb (1) extends more than 1.5, 2 or 2.5 cm from the stop means to the end of each limb (1) that is further removed from the bridge (2).

10. A nasal dilator as claimed in any of the preceding claims, wherein each limb (1) is less than 0.3, 0.25, 0.2 or 0.1 cm thick.

11. A nasal dilator as claimed in any of the preceding claims, further comprising a passageway through which air can flow.

12. A nasal dilator as claimed in claim 11, wherein the passageway is associated with a limb (1) and/or wherein the passageway and/or limb (1) associated with the passageway is made of a flexible material.

13. A nasal dilator as claimed in any of the preceding claims that is less than 1 g, 0.5g or 0.25g in weight.

14. A nasal dilator as claimed in any of the preceding claims that is made of a synthetic plastics selected from nylon, polyethylene, polypropylene, or any combination thereof or from acetal.

15. A nasal dilator as claimed in any of claims 7 to 8, wherein the at least one stop means comprises a grip portion (6).

## Patentansprüche

1. Nasaldilator, der dafür gestaltet ist, in ein einzelnes Nasenloch eingeführt zu werden, wobei der Nasaldilator mindestens zwei gegenüberliegende Glieder (1) umfasst, die auseinandergespannt sind und die dafür gestaltet sind, gleichzeitig in ein einzelnes Nasenloch eingeführt werden zu können, wobei die Glieder (1), sobald sie eingeführt sind, ein Auswärtsdehnen am Weichgewebe des Nasenlochs, welches das Ostium internum umgibt, ausführen, **dadurch gekennzeichnet, dass** die gegenüberliegenden Glieder (1) durch eine elastisch verformbare Brücke (2) verbunden sind, wobei sich die Dicke der Glieder (1) vom dicksten Punkt an der Brücke (2) aus verjüngt.

2. Nasaldilator nach Anspruch 1, wobei der Dilator derart geformt ist, dass mindestens zwei gegenüberliegende Glieder (1) gebildet sind, die durch die verformbare Brücke (2) verbunden sind.

3. Nasaldilator nach Anspruch 1, der aus einer Materiallänge gebildet ist, die eine Kerbstelle umfasst, um welche die Materiallänge gebogen werden kann, sodass die elastisch verformbare Brücke (2) gebildet wird, wobei die Materiallängen, die sich von der Kerbstelle aus erstrecken, die Glieder (1) definieren.

4. Nasaldilator nach einem der vorhergehenden Ansprüche, ferner einen gepolsterten Abschnitt umfassend, der an jedem Glied (1) bereitgestellt ist, wobei der gepolsterte Abschnitt an jedem Glied (1) oder hin zum Ende jeden Gliedes (1) bereitgestellt ist, das am weitesten von der Brücke (2) entfernt ist.

5. Nasaldilator nach einem der vorhergehenden Ansprüche, wobei die Glieder (1) und die elastisch verformbare Brücke (2) dafür gestaltet sind, sich während des Gebrauchs vollständig in der Nasenhöhle zu befinden.

6. Nasaldilator nach Anspruch 5, wobei die elastisch verformbare Brücke (2) aus einem ersten und einem zweiten elastisch verformbaren U-Bogen (9) gebildet ist, die durch einen dritten elastisch verformbaren U-Bogen (10) verbunden sind, wobei die dritte elastisch verformbare Brücke (2) im Verhältnis zur ersten und zweiten verformbaren Brücke (2) umgekehrt liegt.

7. Nasaldilator nach einem der Ansprüche 1 bis 4, ferner mindestens ein Stoppmittel umfassend, das mit mindestens einem Glied (1) verbunden und derart gestaltet ist, dass der Dilator nicht über das Stoppmittel hinaus in den Nasenraum eingeführt werden kann.

8. Nasaldilator nach Anspruch 7, wobei mindestens ein Stoppmittel einstückig mit dem mindestens einen Glied (1) gebildet ist und wobei das Stoppmittel aus einem sich zur Seite erstreckenden Knick (5) in mindestens einem Glied (1) gebildet ist.

9. Nasaldilator nach einem der vorhergehenden Ansprüche, wobei jedes Glied (1) mehr als 1, 1,5, 2 oder 2,5 cm lang ist und, wenn abhängig von Anspruch 7 oder 8, sich jedes Glied (1) um mehr als 1,5, 2 oder 2,5 cm vom Stoppmittel bis zum von der Brücke (2) weiter entfernten Ende jeden Gliedes (1) erstreckt.

10. Nasaldilator nach einem der vorhergehenden Ansprüche, wobei jedes Glied (1) weniger als 0,3, 0,25, 0,2 oder 0,1 cm dick ist.

11. Nasaldilator nach einem der vorhergehenden Ansprüche, ferner einen Durchlassweg umfassend, durch den Luft strömen kann.

12. Nasaldilator nach Anspruch 11, wobei der Durchlassweg mit einem Glied (1) verbunden ist und/oder wobei der Durchlassweg und/oder das Glied (1), das mit dem Durchlassweg verbunden ist, aus einem flexiblen Material hergestellt ist/sind.

13. Nasaldilator nach einem der vorhergehenden Ansprüche, der weniger als 1 g, 0,5 g oder 0,25 g wiegt.

14. Nasaldilator nach einem der vorhergehenden Ansprüche, der aus einem synthetischen Kunststoff hergestellt ist, der aus Nylon, Polyethylen, Polypropylen oder einer Kombination daraus oder aus Acetal ausgewählt ist.

15. Nasaldilator nach Anspruch 7 oder 8, wobei das mindestens eine Stoppmittel einen Griffabschnitt (6) umfasst.

## Revendications

1. Dilatateur nasal configuré pour être inséré dans une seule narine, le dilatateur nasal comprenant au moins deux branches opposées (1) qui sont sollicitées de manière à s'éloigner l'une de l'autre et sont configurées pour pouvoir être insérées simultanément dans une seule narine, dans lequel, une fois que lesdites branches (1) sont insérées, elles exercent une dilatation vers l'extérieur de la narine sur le tissu mou qui entoure l'orifice interne, **caractérisé en ce que** les branches opposées (1) sont raccordées par un pont élastiquement déformable (2), l'épaisseur des branches (1) diminuant progressivement à partir du point le plus épais sur le pont (2).

2. Dilatateur nasal selon la revendication 1, dans lequel le dilatateur est moulé pour former au moins deux branches opposées (1) raccordées par le pont déformable (2).

3. Dilatateur nasal selon la revendication 1, formé par un segment de matériau comprenant une encoche, autour de laquelle le segment de matériau peut être incurvé de manière à former le pont élastiquement déformable (2), les segments de matériau s'étendant à partir de l'encoche définissant les branches (1).

4. Dilatateur nasal selon l'une quelconque des revendications précédentes, comprenant en outre une partie tamponnée formée sur chaque branche (1), dans lequel la partie tamponnée est formée à ou vers l'extrémité de chaque branche (1) la plus éloignée du pont (2).

5. Dilatateur nasal selon l'une quelconque des revendications précédentes, dans lequel les branches (1) et le pont élastiquement déformable (2) sont configurés, en cours d'utilisation, pour pouvoir être entièrement reçus à l'intérieur de la cavité nasale.

6. Dilatateur nasal selon la revendication 5, dans lequel le pont élastiquement déformable (2) est formé d'un premier et d'un deuxième coude en U élastiquement déformables (9) qui sont raccordés par un troisième coude en U élastiquement déformable (10), dans lequel le troisième pont élastiquement déformable (2) est inversé par rapport aux premier et deuxième ponts élastiquement déformables (2).

7. Dilatateur nasal selon l'une quelconque des revendications 1 à 4, comprenant en outre au moins un moyen d'arrêt associé à au moins une branche (1) et configuré de manière que le dilatateur ne puisse être inséré dans la cavité nasale au-delà du moyen d'arrêt.

8. Dilatateur nasal selon la revendication 7, dans lequel au moins un moyen d'arrêt est formé d'une seule pièce avec la au moins une branche (1) et dans lequel le moyen d'arrêt est formé d'une saillie (5) s'étendant latéralement dans au moins une branche (1).

9. Dilatateur nasal selon l'une quelconque des revendications précédentes, dans lequel chaque branche (1) a une longueur supérieure à 1, 1,5, 2 ou 2,5 cm et, lorsqu'il dépend des revendications 7 à 8, chaque branche (1) s'étend sur plus de 1,5, 2 ou 2,5 cm du moyen d'arrêt à l'extrémité de chaque branche (1) la plus éloignée du pont (2).

10. Dilatateur nasal selon l'une quelconque des revendications précédentes, dans lequel chaque branche (1) a une épaisseur inférieure à 0,3, 0,25, 0,2 ou 0,1 cm.

11. Dilatateur nasal selon l'une quelconque des revendications précédentes, comprenant en outre un passage dans lequel l'air peut s'écouler.

12. Dilatateur nasal selon la revendication 11, dans lequel le passage est associé à une branche (1) et/ou dans lequel le passage et/ou la branche (1) associée au passage sont en matériau flexible.

13. Dilatateur nasal selon l'une quelconque des revendications précédentes qui pèse moins de 1 g, de 0,5 g ou de 0,25 g.

14. Dilatateur nasal selon l'une quelconque des revendications précédentes qui est fabriqué en matière plastique synthétique choisie parmi le Nylon, le polyéthylène, le polypropylène ou toute combinaison de ceux-ci ou un acétal.

15. Dilatateur nasal selon l'une quelconque des revendications 7 à 8, dans lequel le au moins un moyen d'arrêt comprend une partie de préhension (6).
